# DEMANDE DE BREVET EUROPEEN

(11) **EP 1 683 511 A1**
(43) Date de publication de la demande: **26.07.2006**
(21) Numéro de dépôt: 05290080.0
(22) Date de dépôt: 13.01.2005
(51) Int. Cl.: A61K 8/34, A61K 8/365, A61K 8/42, A61Q 19/00, A61K 31/17

(54) **Produit squamo-régulateur à base d'urée et son application en cosmétologie**

(71) Demandeur: Laboratoires S.V.R., 91220 Le Plessis Pate (FR)
(72) Inventeur: Besse Renand, BP 56 91072 Bondoufle (FR)
(74) Mandataire: Burtin, Jean-François

(57) **Abrégé**

L'invention a précisément pour objectif de nouvelles compositions dermatologiques et/ou cosmétologiques caractérisées en ce qu'elles contiennent de l'urée en concentration élevée, de l'acide lactique, et éventuellement du glycérol et/ou de l'ichtyol sous forme libre ou sous forme d'un dérivé chimique.

Les compositions dermatologiques et/ou cosmétiques selon l'invention sont destinées au traitement de troubles dermatologiques et en particulier d'hyperkératose.

## Description

La présente invention se rapporte aux nécessités de la vie, et plus particulièrement au domaine des soins de la peau.

L'invention concerne plus particulièrement le domaine de la régénération de l'épiderme et de la suppression des cellules mortes qui contribuent à conférer à la peau un aspect blanchâtre, ou tout au moins dépourvu de couleurs.

L'invention concerne spécifiquement des compositions dermatologiques et/ou cosmétologiques caractérisées par une concentration élevée en urée et par la présence d'acide lactique, d'acide salicylique ou d'acide glycolique dans une émulsion huile dans l'eau.

On sait, depuis de nombreuses années, que l'urée, à concentration relativement élevée, est un agent kératolytique efficace et augmente la reprise de l'eau dans le stratum cornéum. Ceci confère une capacité élevée de liaison de l'eau qui favorise l'utilisation de l'urée en tant qu'agent humidificateur de la peau ou comme agent augmentant la pénétration transdermique de principes actifs.

L'urée joue également un rôle favorable dans le débridement des plaies, dissout les coagulums et facilite l'épithélialisation, lorsqu'elle est utilisée à des concentrations d'au moins 10 % à 15 %. A des concentrations plus élevées, par exemple au-dessus de 40 %, l'urée est protéolytique et trouve un emploi pour le traitement de la destruction des ongles et leur dissolution. En outre, l'urée est employée pour le traitement de la mobilisation des fluides d'oedème, en tant qu'agent diurétique osmotique, ainsi qu'il est exposé dans le brevet US 4,672,078.

Les travaux de la Demanderesse ont montré que l'urée, par ses propriétés hygroscopiques, a un effet sur la rétention d'eau. Elle augmente la capacité de rétention au niveau du stratum corneum. Des expériences effectuées sur la mesure d'impédance cutanée montrent une chute de l'impédance après application d'une préparation à base d'urée, ce qui correspond à une augmentation de la rétention de l'eau.

L'urée a également une action keratolytique qui est plus efficace que celle de l'acide salicylique.

Il était donc nécessaire de mettre au point des préparations cosmétologiques et/ou dermatologiques à base d'urée, dont la concentration en principe actif soit spécialement déterminée pour réaliser une action kératorégulatrice, c'est à dire susceptible de lutter contre les plaques d'hyperkératose et assurer ainsi une action squamorégulatrice, sans entraîner d'effet d'attaque de la peau ou d'effet protéolytique, ni non plus d'effet simplement humidifiant, tel qu'il est obtenu avec des concentrations faibles en urée.

C'est cet équilibre entre des concentrations élevées, nettement lytiques, et des concentrations faibles en urée, simplement hydratantes, qui constitue l'essence de l'invention et qui contribue à conférer aux compositions dermatologiques et/ou cosmétologiques de l'invention des propriétés nouvelles, et jusqu'ici non décrites dans la Littérature.

L'action régulatrice de l'urée est renforcée par la présence d'acide lactique qui confère un pH acide. L'acide lactique se trouve présent à une concentration de 1 à 5 %, et de préférence de 2 à 4 %

L'action régulatrice de l'urée est également renforcée par la présence de glycérol à concentration importante. Le glycérol détruit les cornéocytes dans les cornéodesmosomes et contribue à rompre les liaisons hydrogènes. La teneur en glycérol varie de 2 à 10 % en poids, et plus particulièrement de 4 à 6 %. En outre, l'urée se trouve de préférence sous une forme stabilisée dénommée « cornéosphère », (Kobo) qui lui assure un pouvoir lytique et dénaturant vis-à- vis des cornéocytes et évite sa dégradation.

En définitive, les teneurs en urée dans les préparations dermatologiques et/ou cosmétiques selon l'invention s'échelonnent de 10 à 50 % en poids, et se situent de préférence vers 30 %.

L'effet de l'urée est encore renforcé par l'addition d'ichtyol sous forme libre ou sous forme d'un dérivé chimique, tel que l'ichtyol sulfonate de calcium ou l'ichtyol sulfonate de sodium.

En outre, la littérature enseigne que, pour être efficace, l'urée doit se présenter sous forme d'émulsion huile dans l'eau.

Afin de pouvoir agir sur la peau, l'urée doit être introduite dans un vecteur dont la nature a une influence sur l'efficacité. L'urée est très soluble dans l'eau, donc elle se dissout dans la phase aqueuse d'une émulsion. Si l'urée est introduite dans une émulsion huile dans l'eau- c'est-à-dire si de fines gouttes d'huile sont entourées d'une phase aqueuse externe, elle pénètre rapidement en concentration élevée dans la partie supérieure de la couche cornée. Cependant, dans les couches profondes du stratum corneum et dans les zones où se produit la division cellulaire, elle est peu détectable, même après des temps de contact prolongés. Au contraire, si l'urée se trouve présente sous la forme d'une émulsion Eau/Huile, la phase aqueuse se trouve entourée d'une phase huileuse continue, externe, et la phase aqueuse se répartit de façon homogène dans la totalité de la couche cornée. On la retrouve également dans les zones profondes de l'épiderme, à des concentrations relativement élevées. De ce fait, l'action de l'urée se manifeste d'une manière constante et régulière.

Les compositions dermatologiques et/ou cosmétiques selon l'invention sont présentées sous forme de crèmes, de gels, de baumes et/ou de shampooings. Elles sont parfaitement compatibles avec des agents tensioactifs ioniques, comme le laurith 2-sulfosuccinate disodique. Elles sont également compatibles avec l'addition d'acide salicylique ou d'acide glycolique.

Pour la préparation de shampooings les compositions peuvent être parfumées et on utilisera, à cet effet, des essences naturelles comme l'essence de lavande, l'essence de lavandin ou l'essence de fleurs d'oranger par exemple. On peut également utiliser les principes actifs de ces essences, comme le lavandinol, les ionones, les irones ou le nérolidol.

Les compositions selon l'invention pourront également être additionnées de silicones qui améliorent la sensation de douceur à l'application. Des silicones appropriés sont, par exemple, la diméthicone, la siméthicone ou la hexamethicone.

Les teneurs en silicone peuvent, avantageusement, se situer entre 1 et 2,2 %.

Les compositions selon l'invention se présentent sous forme de crèmes, de laits, de lotions, de gels, de baumes ou de shampooings.

La phase huileuse pourra être constituée :
- d'une huile végétale, comme l'huile d'amandes douces ou l'huile d'avocat, ou bien
- d'une huile minérale, comme l'huile de paraffine
- ou d'un mélange des deux.

Les compositions kérato- régulatrices selon l'invention sont destinées à être appliquées sur la peau, dans les cas d'ichtyose, à raison de 1 à 4 applications par jour, à la dose de 0,2 à 0,6 g par application. La durée du traitement s'étend pendant plusieurs semaines, en considérant qu'un traitement de quatre semaines est une durée nécessaire pour mettre en évidence un effet positif.

Les essais ont été menés pendant au moins 28 jours, en appliquant une fois par jour sur la peau de femmes volontaires, dans une étude en double aveugle randomisée. Ces sujets ont appliqué deux préparations hydratantes, une de chaque côté du corps, sur les zones circonscrites de la peau des jambes qui constituent le modèle optimal de peau sèche.

### EXEMPLES DE PREPARATIONS

### Exemple 1 : Crème à 10 g d'urée

| | g |
|---|---|
| Urée sous forme de Cornéosphères (Kobo) | 10,0 |
| Acide lactique | 2,0 |
| Huile d'amandes douces | 60,0 |
| Ascorbate de palmityle | 0,5 |
| Germaben III | 0,4 |
| Sucrose ester (ester palmitique de sucrose) | 0,4 |
| Eau distillée qsp | 20,0 |

### Exemple 2 : Crème à 30 g d'urée

| | g |
|---|---|
| Urée sous forme de Cornéosphères (Kobo) | 30,0 |
| Acide lactique | 1,0 |
| Glycérol | 10,0 |
| Huile d'avocat | 40,0 g |
| Beurre de karité | 10,0 |
| Parabens | 0,3 |
| Acétate de Tocophéryle | 0,5 |
| Polysorbate 80 | 0,4 |
| Essence de lavandin | 0,3 |
| Eau distillée qsp | 100,0 |

### Exemple 3 : Crème à 50 % d'urée

| | g |
|---|---|
| Urée sous forme de Cornéosphères (Kobo) | 50,0 |
| Eau | 20,0 |
| Conservateur | 0,2 |
| Acide lactique | 1,0 |
| Huile d'avocat | 40,0 |
| Acétate de Tocophéryle | 0,5 |
| Stéarate de polyéthylène glycol | 0,1 |

### Exemple 4 : Crème à l'ichtyol sulfonate de sodium

| | g |
|---|---|
| Urée sous forme de Cornéosphères (Kobo) | 10,0 |
| Acide lactique | 2,5 |
| Glycol | 4,0 |
| Huile d'amandes douces | 20,0 |
| Ascorbate de sodium | 0,40 |
| Parabens | 0,30 |
| Sucrose ester | 0,25 |
| Ichtyol sulfonate de sodium | 0,2 |
| Eau distillée | 80,0 |

### EVALUTATION CLINIQUE DES CREMES SELON L'INVENTION

### LES ESSAIS :

Une étude en double aveugle randomisée chez 20 volontaires à peau sèche et très sèche, concerne l'analyse controlatérale de l'activité hydratante de deux émulsions :
- l'une à phase aqueuse continue d'urée à 10 %,
- l'autre à phase huileuse continue d'urée à 30 %.

Il s'agissait d'évaluer l'effet de chacune de ces préparations à base d'urée au cours de huit heures suivant leur application. Les observations ont été effectuées à la première application (J0) et à la dernière application (J28), après un traitement quotidien de 28 jours. Les évaluations concernaient d'une part les mesures de l'apport d'eau dans les couches superficielles de la peau (Sébumétrie), des pertes d'eau par évaporation/effet occlusif (Téwamétrie)

A la fin de l'essai, la tolérance cutanée aux applications répétées a été contrôlée par l'investigateur dermatologue, et les perceptions subjectives des volontaires ont été relevées.

### LES RESULTATS:

Les résultats indiquent une bonne reconstitution du film hydrolipidique, sans effet occlusif excessif, avec une perception positive des volontaires. La comparaison des émulsions met en évidence un effet hydratant (+66 %) similaire avec H/E et E/H.

Cet effet persiste huit heures après l'application de chacune des émulsions (+ 32 % et + 42 %), avec une rémanence plus favorable à l'émulsion E/H.

A la fin du traitement, l'effet des applications répétées et l'analyse de la persistance confirme l'efficacité hydratante des préparations avec une performance renforcée de l'émulsion E/H. L'analyse de la tolérance cutanée est apparue très satisfaisante.

Ces résultats montrent l'intérêt des émulsions H/E et E/H à base d'urée dans une reconstitution nécessaire et suffisant du film hydrolipidique cutané et justifient l'indication des produits contenant de l'urée dans le traitement des peaux sèches et très sèches.

L'état des peaux sèches et très sèches induit diverses sensations d'inconfort : tiraillements, picotements, prurit, etc... La peau paraît terne, rugueuse, squameuse, avec une perte de souplesse et d'élasticité. La déficience du film hydrolipidique de surface altère le rôle protecteur de la barrière épidermique, déséquilibre les flux de l'eau épidermique et s'accompagne parfois de troubles de la kératinisation.

La principale fonction d'un soin cosmétique, dans le traitement de ces états, est :
- d'assurer une reconstitution nécessaire et suffisante du film hydrolipidique pour restaurer le mécanisme de l'hydratation cutanée,
- rétablir un rôle protecteur et
- favoriser les apaisements correspondants.

Le but de l'étude visait à évaluer l'activité hydratante de deux types d'émulsion par l'analyse des modifications induites sur la peau en fonction du délai suivant l'application.

### LA TOLERANCE:

L'évaluation de la tolérance effectuée dans le contexte de ce test d'usage sous contrôle dermatologique apparaît satisfaisante sans aucun effet indésirable notable.

La bonne tolérance des émulsions testées se confirme dans l'évaluation de la perception subjective des volontaires, avec une moyenne des cotations de
- 9,69 sur 10 pour H/E et
- 9,67 sur 10 pour E/H,
en faveur de l'absence de tiraillements, picotements, prurit, rougeurs ou sensation de brûlures.

### LA DISCUSSION :

Dans l'interprétation des résultats, les produits testés répondent au mode d'action de l'hypothèse suivante :
- à J0, la première application constitue l'apport externe d'un film lipidique protecteur, nécessaire à la restauration du mécanisme d'hydratation ;
- à J28, avec le rétablissement progressif d'une régulation naturelle de l'hydratation, cet apport exogène tend à se limiter à une simple fonction d'entretien.

Ainsi, l'émulsion constitue un apport externe qui vient enrichir au niveau des couches superficielles l'eau libre dont la présence est détectée dans la mesure du Cornéomètre (capacitance). L'apport lipidique complémentaire non absorbé reste plus abondant en surface avec une fonction occlusive transitoire, avant élimination par frottements des vêtements.

Selon cette hypothèse, les modifications induites par les émulsions à l'étude ont permis de restaurer l'effet barrière et la fonction de protection de l'épiderme, de rétablir le mécanisme de régulation des échanges trasépidermiques nécessaire à la synthèse des lipides épidermiques. Le rôle de ces lipides, au niveau intercellulaire, d'une part, et au niveau du film hydrolipidique de surface d'autre part, a déjà été mis en évidence dans l'analyse de la perméabilité des épidermes et ses conséquences sur l'état d'hydratation cutanée.

### LA CONCLUSION :

Dans le cas spécifique de deux formulations, l'étude met en évidence la performance hydratante des deux préparations par rapport à la zone témoin non traitée.

La comparaison controlatérale ne permet pas de distinguer, une heure après application, l'effet des émulsions H/E à 10 % d'urée et E/H à 30 % d'urée, soit un apport hydratant à J28 de + 66 %. Cependant, l'émulsion à phase huileuse continue justifie une rémanence et un effet protecteur renforcés, avec une persistance de l'effet huit heures après application de + 42 %, alors que la phase aqueuse continue représente + 32 %.

Ces performances rémanentes se confirment dans l'effet des applications répétées, avec un bénéfice hydratant cumulé par rapport à l'état initial :
- + 11 % pour la crème à 10 % et
- + 20 % pour la crème à 30 %, 24 heures après la dernière application.

En conséquence, deux émulsions de sens contraire peuvent garantir des performances similaires dans la régénération des mécanismes naturels de l'hydratation cutanée, mais répondre à des contextes d'usage spécifiques.

Face à un problème de peau sèche, ou très sèche, une émulsion H/E apportera une réponse à visée plus cosmétologique et une émulsion E/H plus « médicalisée ».

Ainsi, dans cet axe, la formulation de « crème », avec 10 % ou 30 % d'urée, vise un effet kératorégulateur complémentaire de la fonction hydratante.

## Revendications

1. Compositions cosmétologiques et/ou dermatologiques squamo-régulatrices à base d'urée,
**caractérisées en ce qu'**elles contiennent de 10 à 50 % d'urée.

2. Compositions cosmétologiques et/ou dermatologiques squamo-régulatrices selon la revendication 1,
dans lesquelles la teneur en urée se situe vers 30 %.

3. Compositions cosmétologiques et/ou dermatologiques squamo-régulatrices selon la revendication 1 ou la revendication 2,
dans lesquelles l'urée se trouve sous une forme stabilisée, dénommées « Cornéosphères ».

4. Compositions cosmétologiques et/ou dermatologiques squamo-régulatrices selon l'une des revendications 1 à 3,
dans lesquelles l'urée est également additionnée d'acide lactique.

5. Compositions cosmétologiques et/ou dermatologiques squamo-régulatrices selon l'une des revendications 1 à 4,
dans lesquelles l'urée est additionnée en outre de glycérol.

6. Compositions cosmétologiques et/ou dermatologiques squamo-régulatrices selon l'une des revendications 1 à 5,
dans lesquelles l'urée est additionnée en outre d'ichtyol sous forme libre ou sous forme d'un dérivé chimique.

7. Compositions cosmétologiques et/ou dermatologiques squamo-régulatrices selon l'une des revendications 1 à 6,
dans lesquelles le dérivé chimique de l'ichtyol est choisi parmi l'ichtyol sulfonate de calcium et l'ichtyol sulfonate de sodium.

8. Compositions cosmétologiques et/ou dermatologiques squamo-régulatrices selon l'une des revendications 1 à 7,
dans lesquelles on ajoute un agent émulsionnant du type agent tensioactif ionique.

9. Compositions cosmétologiques et/ou dermatologiques squamo-régulatrices selon l'une des revendications 1 à 8,
dans lesquelles les compositions sont parfumées par des essences naturelles ou par des principes actifs d'essences naturelles.

10. Compositions cosmétologiques et/ou dermatologiques squamo-régulatrices selon l'une des revendications 1 à 9,
**caractérisées en ce qu'**elles sont additionnées de silicones et, par exemple la diméthicone, la siméthicone ou l'hexadimethicone.

11. Compositions cosmétologiques et/ou dermatologiques squamo-régulatrices selon l'une des revendications 1 à 10,
**caractérisées en ce qu'**elles sont sous la forme d'une émulsion eau dans l'huile (E/H) ou huile dans l'eau (H/E).

12. Compositions cosmétologiques et/ou dermatologiques squamo-régulatrices selon l'une des revendications 1 à 11,
**caractérisées en ce qu'**elles sont présentées sous la forme de crèmes, de lotions, de laits, de gels, de baumes ou de shampooings.

13. Utilisation des compositions cosmétologiques et/ou dermatologiques selon l'une des revendications 1 à 12,
en vu d'assurer un effet squamo-régulateur en luttant contre les plaques d'hyperkératose.

## Revendications modifiées

### Revendications modifiées conformément à la règle 86(2) CBE.

**1.** Compositions cosmétologiques et/ou dermatologiques squamo-régulatrices à base d'urée,
**caractérisées en ce qu'**elles contiennent
- de 10 à 50 % d'urée, l'urée se trouvant encapsulée dans des nanoparticules protectrices, dénommées « Cornéosphères » (Kobo).
- de l'acide lactique
- et du glycérol

**2.** Compositions cosmétologiques et/ou dermatologiques squamo-régulatrices selon la revendication 1,
dans lesquelles la teneur en urée se situe vers 30 %.

**3.** Compositions cosmétologiques et/ou dermatologiques squamo-régulatrices selon l'une des revendications 1 ou 2,
**caractérisée en ce qu'**elles contiennent en outre de l'ichtyol, sous forme libre ou sous forme d'un dérivé chimique.

**4.** Compositions cosmétologiques et/ou dermatologiques squamo-régulatrices selon l'une des revendications 1 à 3,
dans lesquelles le dérivé chimique de l'ichtyol est choisi parmi l'ichtyol sulfonate de calcium et l'ichtyol sulfonate de sodium.

**5.** Compositions cosmétologiques et/ou dermatologiques squamo-régulatrices selon l'une des revendications 1 à 4,
dans lesquelles on ajoute un agent émulsionnant du type agent tensioactif ionique.

**6.** Compositions cosmétologiques et/ou dermatologiques squamo-régulatrices selon l'une des revendications 1 à 5,
dans lesquelles les compositions sont parfumées par des essences naturelles ou par des principes actifs d'essences naturelles.

**7.** Compositions cosmétologiques et/ou dermatologiques squamo-régulatrices selon l'une des revendications 1 à 6,
**caractérisées en ce qu'**elles sont additionnées de silicones et, par exemple la diméthicone, la siméthicone ou l'hexadimethicone.

**8.** Compositions cosmétologiques et/ou dermatologiques squamo-régulatrices selon l'une des revendications 1 à 7,
**caractérisées en ce qu'**elles sont sous la forme d'une émulsion eau dans l'huile (E/H) ou huile dans l'eau (H/E).

**9.** Compositions cosmétologiques et/ou dermatologiques squamo-régulatrices selon l'une des revendications 1 à 8,
**caractérisées en ce qu'**elles sont présentées sous la forme de crèmes, de lotions, de laits, de gels, de baumes ou de shampooings.

**10.** Utilisation des compositions cosmétologiques et/ou dermatologiques selon l'une des revendications 1 à 9,
en vu d'assurer un effet squamo-régulateur par applications répétées sur la peau.
